# EUROPÄISCHE PATENTSCHRIFT

(11) **EP 1 140 870 B1**
(45) Veröffentlichungstag und Bekanntmachung des Hinweises auf die Patenterteilung: **21.01.2004**
(21) Anmeldenummer: 99963558.4
(22) Anmeldetag: 16.12.1999
(51) Int. Cl.: C07D 251/62

(54) **VERFAHREN ZUR REINIGIUNG VON MALAMIN**
METHOD FOR PURIFYING MELAMINE
PROCEDE DE PURIFICATION DE MELAMINE

(30) Priorität: 23.12.1998 AT 216698
(43) Veröffentlichungstag der Anmeldung: 10.10.2001
(73) Patentinhaber: Agrolinz Melamin GmbH, A-4021 Linz (AT)
(72) Erfinder: COUFAL, Gerhard, A-4060 Leonding (AT)
(74) Vertreter: Gross, Felix
(86) Internationale Anmeldenummer: PCT/EP1999/010008
(87) Internationale Veröffentlichungsnummer: WO 2000/039107

(56) Entgegenhaltungen:
- WO-A-96/20182
- WO-A-96/20183
- WO-A-96/23778
- US-A- 5 514 797

## Beschreibung

Die Erfindung betrifft ein Verfahren zur Reinigung von Melamin, bei dem festes Melamin unter Ammoniakdruck nahe dem Schmelzpunkt verweilen gelassen wird.

Melamin wird bevorzugt durch Pyrolyse von Harnstoff hergestellt, wobei sowohl Niederdruckverfahren als auch Hochdruckverfahren, wie sie beispielsweise in "Ullmann's Encyclopedia of Industrial Chemistry, Vol A 16, 5^{th} ed (1990), Seiten 171-185" beschrieben sind, zur Anwendung kommen können. Das bei der Melaminsynthese anfallende Rohmelamin enthält je nach Herstellverfahren etwa 94-98 Gew. % Melamin, sowie insbesondere Melam, Melem, Melon, Ureidomelamin, Ammelin und Ammelide als, wesentliche Verunreinigungen und muß für anspruchsvollere Anwendungsgebiete durch zusätzliche Verfahrensschritte, wie z.B. Umkristallisieren, oder durch spezielle Verfahrensführung bei der Isolierung, wie z.B. langsames Abkühlen, wie beispielsweise in WO96/20182 beschrieben, durch spezielle Temperaturführung vor der Verfestigung, wie beispielsweise in WO96/23778 beschrieben, oder durch Verweilen des festen Melamins unter Ammoniakdruck, wie beispielsweise in WO96/20183 beschrieben, bzw. durch eine Kombination dieser Verfahrensschritte weiter gereinigt werden.

Es stellte sich jedoch weiterhin die Aufgabe, neue Verfahrensvarianten zu finden, die zu einer weiteren Erhöhung der Melaminreinheit führen, bzw. reineres Melamin auf einfachere Weise, gegebenenfalls auch bei weniger hohen Drücken, zu erhalten.

Unerwarteterweise wurde gefunden, daß beim Verweilen von Melamin unter Ammoniakdruck sowohl bei Temperaturen weit unterhalb des Schmelzpunktes als auch bei Temperaturen knapp oberhalb des Schmelzpunktes mehr Verunreinigungen vorliegen oder gebildet werden, als beim Verweilen bei Temperaturen im Festbereich knapp unterhalb des Schmelzpunktes.

Gegenstand der Erfindung ist demnach ein Verfahren zur Reinigung von Melamin, dadurch gekennzeichnet, daß verunreinigtes Melamin in einen Temperaturbereich T_{T}, der über 325 °C und unterhalb des vom jeweils herrschenden Ammoniakdruck abhängigen Schmelzpunktes von Melamin liegt, gebracht und bei einem Ammoniakdruck von 1 bis 150 bar für die Dauer von 1 Minute bis zu 20 Stunden in diesem Temperaturbereich gehalten wird, wobei das Melamin in diesem Temperaturbereich als Feststoff vorliegt, worauf in beliebiger Reihenfolge entspannt und gewünschtenfalls auf Raumtemperatur abgekühlt und reines Melamin in fester Form erhalten wird.
Das erfindungsgemäße Verweilen des festen Melamins (Tempern) erfolgt bevorzugt in einem Temperaturbereich T_{T} zwischen 330 °C (bzw. besonders bevorzugt zwischen 335 °C) und dem vom jeweiligen Ammoniakdruck abhängigen Schmelzpunkt des Melamins.
Die Temperatur kann während des Tempems konstant gehalten werden, sie kann jedoch auch innerhalb des oben definierten Temperaturbereiches T_{T} verändert werden. So kann die Temperatur beispielsweise kontinuierlich oder diskontinuierlich innerhalb der Grenzen des Temperaturbereiches T_{T} abgesenkt bzw. erhöht werden.

Der Druck beim Tempern liegt erfindungsgemäß bevorzugt zwischen 5 und 100 bar, besonders bevorzugt zwischen 10 und 50 bar. Die Verweilzeit während des erfindungsgemäßen Tempems liegt bevorzugt bei 5 min bis 10 h, besonders bevorzugt bei 30 min bis 5 h. Die Verweilzeit richtet sich vor allem nach dem gewünschten Reinigungsgrad des Melamins, je länger getempert wird, desto reineres Melamin wird erhalten. Die Verweilzeit ist dabei von den jeweiligen Prozeßbedingungen abhängig. Zur Erzielung eines bestimmten Reinheitsgrades ist demnach bei höheren Temperaturen sowie bei höheren Drücken jeweils eine kürzere Verweilzeit ausreichend.

Das erfindungsgemäße Reinigungsverfahren (Tempern) kann dabei bevorzugt derart durchgeführt werden, daß entweder verunreinigtes festes Melamin durch Erhitzen, bzw. verunreinigtes flüssiges Melamin durch Abkühlen in den Temperaturbereich (Temperbereich) T_{T} gebracht wird und dort verweilt.

Nach Beendigung des Temperns kann, je nach den technischen Gegebenheiten, zuerst abgekühlt und dann entspannt, oder in umgekehrter Reihenfolge zuerst entspannt und danach abgekühlt werden. Die Durchführung dieser Schritte kann gegebenenfalls in einer weiteren Reaktionsapparatur erfolgen. Melamin wird dabei bevorzugt auf Raumtemperatur, beispielsweise mit Hilfe von Wärmetauschern, einfaches Entfernen des Heizmediums oder durch Mischen mit kalten Gasen, abgekühlt. Melamin wird durch das erfindungsgemäße Verfahren in kristalliner Form bzw. als Pulver erhalten und weist insbesondere einen deutlich reduzierten Gehalt an Melam, Melem und Ammelid auf, der teilweise sogar unter der Nachweisgrenze liegt.
Das erfindungsgemäße Verfahren kann sowohl diskontinuierlich als auch kontinuierlich durchgeführt werden.
Die erfindungsgemäße Melaminreinigung eignet sich insbesondere im Anschluß an einen Prozeß zur Herstellung von Melamin, insbesondere im Anschluß an einen beliebigen Hochdruckprozeß zur Herstellung von Melamin aus Harnstoff, bei dem das Melamin zunächst in flüssiger Form als Schmelze anfällt. Dabei ist vorteilhaft, die ammoniakhältige Melaminschmelze vor dem Verfestigen insbesondere durch Zufuhr von weiterem Ammoniak auf etwa 1 bis 50 °C über den druckabhängigen Schmelzpunkt des Melamins zu kühlen. Die anschließende Verfestigung erfolgt bevorzugt in einem Wirbelbett durch Inkontaktbringen mit kalten festen Inertstoffen oder festem Melamin. Das erfindungsgemäße Verfahren kann vorteilhaft auch im Anschluß an andere beliebige Aufarbeitungsschritte von Hochdruckverfahren durchgeführt werden. Diese Aufarbeitungsschritte beinhalten insbesondere:
a) Abtrennen des bei der Melaminsynthese erhaltenen NH₃/CO₂-Gasgemisches (Offgase) vom flüssigen Melamin,
b) Reduktion des im Melamin gelösten CO₂ durch Einbringen von NH₃ (Strippen),
c) Verweilenlassen des flüssigen Melamins in Anwesenheit von Ammoniak (Aging),
d) Abkühlen und Verfestigen des Melamins, beispielsweise mit Wasser, mit wäßrigen melaminhaltigen Lösungen oder Suspensionen, mit kalten Gasen, wie gasförmigem Ammoniak, mit flüssigem Ammoniak oder mit kalten festen Inertstoffen oder festem Melamin gemäß WO99/38852, beispielsweise in einem Wirbelbett. Die Melaminschmelze kann beispielsweise gemäß WO97/20826 in einen Abkühlbehälter eingesprüht werden, in dem eine Ammoniakatmosphäre vorliegt.

Das erfindungsgemäße Reinigungsverfahren wird dabei im Anschluß an den Verfestigungsschritt durchgeführt, wobei die Anzahl der durchgeführten anderen vorangegangenen Aufarbeitungsschritte nach den jeweiligen Gegebenheiten variieren kann. Die Aufarbeitung des durch ein Hochdruckverfahren aus Harnstoff gewonnen Melamins bis zum Verfestigungsschritt kann somit jede beliebige Kombination oder auch nur einen dieser Schritte umfassen.

Gemäß Erfindung ist die Reinigung von Melamin beliebiger Reinheit möglich. Die Reinheit eines Rohmelamins aus einem Melaminherstellungsprozeß beträgt je nach angewandtem Melaminprozeß beispielsweise etwa 94 - 98 Gew. %, wobei das Melamin vor allem mit Melam, Melem und Ammelid verunreinigt- ist. Es ist jedoch auch möglich, höher verunreinigte Melamine, aber auch auch bereits mit anderen Reinigungsverfahren vorgereinigte Melamine mit höherer Reinheit erfindungsgemäß weiter zu reinigen. Dabei ist es möglich, je nach angewandtem Druck, Temperatur, Verweilzeit und Ausgangsreinheit, Melamin mit einer Reinheit von bis zu über 99,9 Gew. %, teilweise über 99,99 Gew. % zu erhalten.

### Beispiele

Als Ausgangsmaterial wurde jeweils ein aus einer Hochdruck-Pilotanlage zur Melaminherstellung aus Harnstoff stammendes Melamin mit einer Reinheit von 98,6 Gew. %, das insbesondere mit 1,3 Gew. % Melam sowie etwa 0,1 Gew. % weiteren Nebenprodukten, wie z.B. Melem oder Ammelid, verunreinigt war, eingesetzt (entsprechend Beispiel A in Tabelle 1).
Die analytische Bestimmung von Melam, Melem und Ammelid erfolgte mittels HPLC.

### Beispiele 1 bis 5

In einen Laborautoklaven wurde Melamin sowie die zur Erzielung des gewünschten Druckes notwendige Ammoniakmenge eingebracht. Anschließend wurde der Autoklav auf eine Temperatur von 340 °C bzw. 330 °C gebracht und beim jeweiligen Druck 60 min verweilen gelassen. Dann wurde der Autoklav schnell mit Wasser auf 280 °C abgekühlt, entspannt und das Melamin mittels HPLC analysiert. Die jeweiligen Drücke und Temperaturen beim Tempern, sowie der Gehalt an Nebenprodukten (Melam, Metern, Ammelid) des Ausgangsmelamins (Beispiel A) sowie des Melamins nach dem Tempern (Beispiele 1-5) sind in Tabelle 1 zusammengestellt.

**Tabelle 1:**

| Melamin nach 60 min Tempern | | | | | |
|---|---|---|---|---|---|
| Beispiel | Druck (bar) | Temp. (°C) | Melam (ppm) | Melem (ppm) | Ammelid (ppm) |
| A* | - | - | 12630 | 431 | 155 |
| 1 | 10 | 340 | 323 | 177 | - |
| 2 | 20 | 340 | 443 | 319 | - |
| 3 | 40 | 340 | 131 | 252 | 39 |
| 4 | 70 | 340 | 58 | 122 | - |
| 5 | 100 | 330 | 105 | 162 | - |

| | | | | | |
|---|---|---|---|---|---|
| Nachweisgrenze 2 ppm * Ausgangsmelamin | | | | | |

## Patentansprüche

1. Verfahren zur Reinigung von Melamin, **dadurch gekennzeichnet, daß** verunreinigtes Melamin in einen Temperaturbereich T_{T} der über 325 °C und unterhalb des vom jeweils herrschenden Ammoniakdruck abhängigen Schmelzpunktes von Melamin liegt, gebracht und bei einem Ammoniakdruck von 1 bis 150 bar für die Dauer von 1 Minute bis zu 20 Stunden in diesem Temperaturbereich gehalten wird, wobei das Melamin in diesem Temperaturbereich als Feststoff vorliegt, worauf in beliebiger Reihenfolge entspannt, abgekühlt und reines Melamin in fester Form erhalten wird.

2. Verfahren nach Anspruch 1, **dadurch gekennzeichnet, daß** verunreinigtes flüssiges Melamin in den Temperaturbereich T_{T} gebracht wird.

3. Verfahren nach Anspruch 1, **dadurch gekennzeichnet, daß** verunreinigtes festes Melamin in den Temperaturbereich T_{T} gebracht wird.

4. Verfahren nach Anspruch 1, **dadurch gekennzeichnet, daß** das verunreinigte Melamin in einen Temperaturbereich, der zwischen 330 °C und unterhalb des vom jeweils herrschenden Ammoniakdruck abhängigen Schmelzpunktes von Melamin liegt, gebracht wird.

5. Verfahren nach Anspruch 1, **dadurch gekennzeichnet, daß** der Ammoniakdruck zwischen 5 und 100 bar liegt.

6. Verfahren nach Anspruch 1, **dadurch gekennzeichnet, daß** das Melamin für eine Dauer von 5 Minuten bis 10 Stunden im Temperaturbereich T_{T} gehalten wird.

7. Verfahren nach Anspruch 1, **dadurch gekennzeichnet, daß** das Verfahren an einen Prozeß zur Herstellung von Melamin angeschlossen wird.

8. Verfahren nach Anspruch 1, **dadurch gekennzeichnet, daß** das Verfahren an einen Hochdruckprozeß zur Herstellung von Melamin aus Harnstoff angeschlossen wird, bei dem flüssiges Melamin gegebenenfalls nach Verweilenlassen in Anwesenheit von Ammoniak (Aging) in einem Wirbelbett durch Inkontaktbringen mit kalten festen Inertstoffen oder festem Melamin verfestigt und anschließend in einem Temperbereich T_{T} der über 325 °C und unterhalb des vom jeweils herrschenden Ammoniakdruck abhängigen Schmelzpunktes vom Melamin liegt, bei einem Ammoniakdruck von 1 bis 150 bar für die Dauer von 1 Minute bis zu 20 Stunden in diesem Temperaturbereich gehalten wird, worauf in beliebiger Reihenfolge entspannt, abgekühlt und reines Melamin in fester Form erhalten wird.

## Claims

1. Method for purifying melamine, **characterized in that** impure melamine is brought into a temperature range T_{T} above 325°C and lower than the melting point of melamine which depends on the respective prevailing ammonia pressure and is kept in this temperature range at an ammonia pressure of from 1 to 150 bar for the duration of from 1 minute to 20 hours, the melamine being present in this temperature range as a solid, after which, in any desired order, the pressure is relieved, cooling is effected and pure melamine is obtained in solid form.

2. Method according to claim 1, **characterized in that** impure liquid melamine is brought into the temperature range T_{T}.

3. Method according to claim 1, **characterized in that** impure solid melamine is brought into the temperature range T_{T}.

4. Method according to claim 1, **characterized in that** the impure melamine is brought into a temperature range which is between 330°C and lower than the melting point of melamine which depends on the respective prevailing ammonia pressure.

5. Method according to claim 1, **characterized in that** the ammonia pressure is between 5 and 100 bar.

6. Method according to claim 1, **characterized in that** the melamine is kept in the temperature range T_{T} for a duration of from 5 minutes to 10 hours.

7. Method according to claim 1, **characterized in that** the method is carried out after a process for the production of melamine.

8. Method according to claim 1, **characterized in that** the method is carried out after a high-pressure process for the production of melamine from urea, in which liquid melamine, optionally after being allowed to dwell in the presence of ammonia (ageing), is solidified in a fluidized bed by being brought into contact with cold solid inert substances or solid melamine and is then kept in a temperature range T_{T} above 325°C and lower than the melting point of melamine which depends on the respective prevailing ammonia pressure, at an ammonia pressure of from 1 to 150 bar for the duration of from 1 minute to 20 hours, after which, in any desired order, the pressure is relieved, cooling is effected and pure melamine is obtained in solid form.

## Revendications

1. Procédé de purification de la mélamine, **caractérisé en ce que** la mélamine impure est amenée dans une plage de température T_{T} supérieure à 325°C et inférieure au point de fusion de la mélamine qui dépend de la pression d'ammoniac qui règne dans chaque cas, et est maintenue dans cette plage de température à une pression d'ammoniac de 1 à 150 bars pendant une période de 1 minute à 20 heures, la mélamine se trouvant sous forme d'un solide dans cette plage de température, après quoi, dans un ordre quelconque, on relâche la pression, on refroidit et on obtient la mélamine pure sous forme solide.

2. Procédé selon la revendication 1, **caractérisé en ce que** la mélamine liquide impure est amenée dans la plage de température T_{T}.

3. Procédé selon la revendication 1, **caractérisé en ce que** la mélamine solide impure est amenée dans la plage de température T_{T}.

4. Procédé selon la revendication 1, **caractérisé en ce que** la mélamine impure est amenée dans une plage de température qui est comprise entre 330°C et en dessous du point de fusion de la mélamine qui dépend de la pression d'ammoniac qui règne dans chaque cas.

5. Procédé selon la revendication 1, **caractérisé en ce que** la pression d'ammoniac est comprise entre 5 et 100 bars.

6. Procédé selon la revendication 1, **caractérisé en ce que** la mélamine est maintenue dans la plage de température T_{T} pendant une période de 5 minutes à 10 heures.

7. Procédé selon la revendication 1, **caractérisé en ce que** le procédé est suivi d'un processus de préparation de la mélamine.

8. Procédé selon la revendication 1, **caractérisé en ce que** le procédé est suivi d'un processus à haute pression de préparation de la mélamine à partir de l'urée, dans lequel la mélamine liquide, éventuellement après l'avoir laissée en présence d'ammoniac (vieillissement), est solidifiée dans un lit fluidisé par mise en contact avec des substances inertes solides froides ou de la mélamine solide, et est ensuite maintenue dans une plage de température T_{T} supérieure à 325°C et inférieure au point de fusion de la mélamine qui dépend de la pression d'ammoniac qui règne dans chaque cas, à une pression d'ammoniac de 1 à 150 bars pendant une période de 1 minute à 20 heures, dans cette plage de température, après quoi, dans un ordre quelconque, on relâche la pression, on refroidit et on obtient la mélamine pure sous forme solide.
